(19) Europäisches Patentamt
European Patent Office
Office européen des brevets

(11) Publication number: **0 134 259 B1**

(12) **EUROPEAN PATENT SPECIFICATION**

(45) Date of publication of patent specification: **27.05.92**

(51) Int. Cl.[5]: **C07C 241/02**, C01B 21/16, C01B 21/09

(21) Application number: **83107623.7**

(22) Date of filing: **02.08.83**

(54) **Improved process for preparing hydrazines.**

(43) Date of publication of application:
**20.03.85 Bulletin 85/12**

(45) Publication of the grant of the patent:
**27.05.92 Bulletin 92/22**

(84) Designated Contracting States:
**AT BE DE FR GB IT NL**

(56) References cited:
**EP-A- 0 027 645**
**FR-A- 2 396 743**
**GB-A- 824 357**
**GB-A- 1 561 146**

(73) Proprietor: **Osborg, Hans**
**P.O. Box 152 80 Longview Road**
**Port Washington, N.Y. 11050(US)**

(72) Inventor: **Osborg, Hans**
**P.O. Box 152 80 Longview Road**
**Port Washington, N.Y. 11050(US)**

(74) Representative: **Froud, Clive et al**
**Elkington and Fife Prospect House 8 Pembroke Road**
**Sevenoaks, Kent TN13 1XR(GB)**

EP 0 134 259 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing hydrazine and various hydrocarbyl-substituted hydrazines.

Hydrazine, alkyl-substituted hydrazines, particularly unsymmetrical dimethylhydrazine (UDMH), and phenyl-substituted hydrazines, are important commercial compounds having a wide range of utilities, such as pharmaceuticals, fuels, agricultural products and intermediates in the preparation of blowing agents.

A number of processes have been used heretofore for the preparation of hydrazine and its derivatives. For example, the Raschig process is a commercial synthesis of hydrazine from chloramine and ammonia in aqueous solution. Intially, sodium hypochlorite is reacted with an excess of ammonia to form chloramine, with sodium hydroxide being produced as a by-product. The chloramine then reacts with ammonia to form hydrazine. In the first stage of the process, chloramine is formed rapidly. However, in the second stage the reaction of chloramine with ammonia is slow and requires heat for completion. The rate of formation of the hydrazine product increases with temperature. As a side reaction, the hydrazine reacts with the starting chloramine to form ammonium chloride and nitrogen. In order to avoid an undesirably high rate of hydrazine decomposition by this undesirable side reaction, the process must be carried out at high temperatures (about 130 ° C) and with a large excess of ammonia (20:1 to 30:1) to minimize the reaction of hydrazine product with chloramine reactant. The desired hydrazine product is produced in relatively low concentration (generally from 1% to 3%) in the final reaction mixture, which contains a considerable amount of water, making recovery of anhydrous hydrazine from the mixture rather costly.

The Olin process (Kobe et al., Advances in Petroleum Chemistry and Refining, Vol. 2, Interscience Pub. Inc., New York, N.Y., 1959, Chapter 9) is a modification of the Raschig process employing anhydrous ammonia, which is injected under pressure into an aqueous chloramine solution. Due to the heat of dilution, the temperature of the reaction mixture is raised to about 130 ° C, the optimum temperature for the reaction of ammonia with chloramine. However, additional heat must be provided from an outside fuel source to carry the reaction to completion and to separate the relatively small concentration of hydrazine from the rather large volume of ammonia in subsequent distillation steps. Further energy is required to remove sodium chloride and sodium hydroxide by-products and to recover the hydrazine. As in the Raschig process, the hydrazine is recovered as the monohydrate. To obtain the pure anhydrous product substantial additional energy is required to drive off the chemically bound water.

The Schestakoff method is based on the degradation of urea by sodium hypochlorite to produce hydrazine. The reaction is analogous to the Hoffmann preparation of primary amines from amides. In the process, a cold aqueous solution of urea and sodium hydroxide is added to a cold aqueous solution of sodium hypochlorite. The heat of reaction increases the temperature to 100 ° C, at which the reaction takes place at a relatively fast rate. Large quantities of steam must be used in the preparation of the urea solution (43% solution) to offset the huge endotherm of solution. The product, as in the aforementioned commercial processes, is hydrazine monohydrate in rather low concentration (about 3%). Additional energy is required to concentrate, convert the hydrate and fractionate the final hydrazine product. Excessive quantities of alkali and alkali salts are produced as by-products (ratio about 12:1 of by-products to $N_2H_4$ thus manufactured, by weight), which are not reusable in the process.

The Bergbau or Bayer process is yet another commercial procedure for the production of hydrazine. The energy requirements of the Bergbau process area not as great as in the above-described commercial processes. In this process, ammonia is reacted with chlorine in the presence of a ketone to form an intermediate diazocyclopropane or ketazine. The intermediate is then hydrolyzed to the hydrazine hydrate and the latter converted to the desired anhydrous product. The energy requirements for recovery of the hydrazine are approximately the same as for the commercial processes previously described, which involve recovery from aqueous reaction mixtures.

It can thus be seen that the most well known and widely practiced hydrazine manufacturing processes produce a hydrate of hydrazine, which requires substantial energy for recovery of the anhydrous product.

In view of constantly rising energy costs, it is of paramount importance to minimize the use of energy in excess of thermodynamic requirements needed to complete a given reaction. In considering the overall energy requirements of any chemical manufacturing process, the energy needed for the production of raw materials and auxiliary chemicals must also be taken into accout. Thus, in the case of hydrazine, the energy requirement for providing $NH_3$, $Cl_2$, NaOCl, urea, NaOH etc., is a significant factor in determining the degree of gain or loss in the energy required to make the final product. Similarly, the weight ratio of by-products versus the desired end-product must be evaluated as another important factor in the overall efficiency of a process. Considered in this light, the above-described prior art methods for the production of hydrazine are decidedly inefficient, especially when it is considered that fifty-eight kcal./mole of product

hydrazine are lost from the system upon the formation of hydrazine hydrate.

A process currently used for the commercial production of UDHM involves nitrosation of the sulfuric acid salt of dimethylamine using sodium nitrite to obtain dimethylnitrosamine, which is reduced to the desired product. In addition to suffering from many of the inefficiencies noted above with respect to the hydrazine prosesses, the dimethylnitrosamine produced as an intermediate in this process is a known carcinogen and poses a potential hazard not only to personnel operating the process but to the environment as well. Because of this potential hazard, the Occupational Safety and Health Administration (OSHA) has implemented strict rules regulating manufacturing processes in which any nitrosamine is used or produced.

Many of the inefficiencies and the hazards inherent in the prior art have been overcome by the process described in U.S. Patent No. 4,286,108, which produces hydrazine and hydrocarbyl-substituted hydrazines by reacting a tertiary hydrazinium halide with a compound selected from the group consisting of an alkali metal amide, an alkaline earth metal amide, a hydrocarbyl-substituted alkali metal amide or a hydrocarbyl-substituted alkaline earth metal amide, in the presence of a non-aqueous inert carrier.

One of the reactants used in the preparation of the tertiary hydrazinium halide according to the process of the '108 patent is chloramine produced by reacting ammonia gas with chlorine. A by-product of this reaction is ammonium chloride which has a tendency to clog the reactant delivery lines, and generally hampers operation of the process.

## SUMMARY OF THE INVENTION

In accordance with the present invention, there is provided a process for preparing anhydrous hydrazine and its hydrocarbyl-substituted derivatives, which comprises reacting an alkali metal amide, an alkaline earth metal amide, a hydrocarbyl-substituted alkali metal amide or a hydrocarbyl-substituted alkaline earth metal amide with chlorine to produce chloramine or a hydrocarbyl-substituted chloramine without co-production of ammonium chloride; reacting the chloramine thus produced with an at least equimolar amount of a tertiary amine to produce a tertiary hydrazinium chloride; and reacting the tertiary hydrazinium chloride with an alkali metal amide, an alkaline earth metal amide, a hydrocarbyl-substituted alkali metal amide or a hydrocarbyl-substituted alkaline earth metal amide under anhydrous conditions to produce the desired product.

The term "hydrocarbyl" as used herein is intended to signify any monovalent radical obtained upon removal of a hydrogen atom from a parent hydrocarbon. Representative of hydrocarbyl radicals are alkyl of 1 to 25 carbon atoms, inclusive, such as methyl, ethyl, propyl, butyl, pentyl, hexyl, heptyl, octyl, nonyl, undecyl, decyl, dodecyl, octadecyl, nonadecyl, eicosyl, heneicosyl, docosyl, tricosyl, tetracosyl, pentacosyl, and the isomeric forms thereof; aryl or 6 to 25 carbon atoms, inclusive, such as phenyl, tolyl, xylyl, napthyl, biphenyl and tetraphenyl; aralkyl of 7 to 25 carbon atoms, inclusive, such as benzyl, phenethyl, phenpropyl, phenbutyl, phenhexyl and napthoctyl; cycloalkyl of 3 to 8 carbon atoms, inclusive, such as cyclopropyl, cyclobutyl, cyclohexyl, cycloheptyl and cyclooctyl.

The term "alkali metal", as used herein in, is intended to include lithium, sodium, potassium, rubidium and cesium.

The term "alkaline earth metal", as used herein, is intended to include magnesium, calcium, barium and strontium.

By "under anhydrous reaction conditions" is meant in the presence of less than one weight percent water, preferably less than 0.1%. Illustrative of suitable carriers are dried kerosene (preferably of low sulphur content and freshly distilled), trialkylamines, such as tripropylamine and tributylamine, carbon tetrachloride, alkyl ethers, and mixtures thereof. It has been found that the use of kerosene as the reaction medium in each step of the process markedly enhances the yield of hydrazine, or hydrocarbyl-substituted hydrazines. All of the by-products are commercially useful substances which may be conveniently recovered. Most of these substances may be recirculated to provide starting materials for certain steps in the process, or to serve as raw materials for the production of said starting materials.

As will be apparent from the following detailed description, the process of the present invention has all of the advantages of the process covered by US Patent No 4,286,108, in that the process requires no specialized equipment, the cost of reactants is minimized and the desired product is readily obtained using standard recovery techniques. In addition, anhydrous hydrazine and hydrocarbyl-substituted hydrazines may be obtained in relatively high concentrations (on the order of 25% to 50%) in the reaction mixture. Another distinct advantage of the process of the present invention is that it avoids the co-production of ammonium chloride which may interfere with the efficient operation of the process.

Another notable advantage of the present invention is that it provides a relatively safe procedure for the preparation of hydrocarbyl-substituted hydrazines, especially UDMH. Unlike the methods currently in

practice for producing such products, the process of the present invention is carried out under conditions which offer little or no threat of harm to operating personnel or the environment.

DETAILED DESCRIPTION OF THE INVENTION

The process of the present invention is carried out according to the following general reaction scheme:

A.

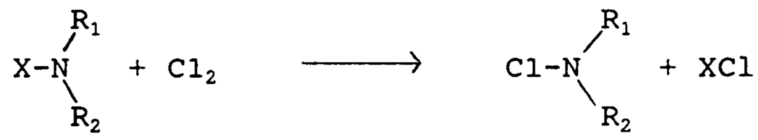

(I) (II) (III)

B.

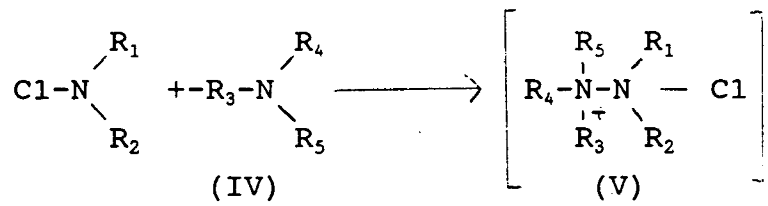

(IV) (V)

C.

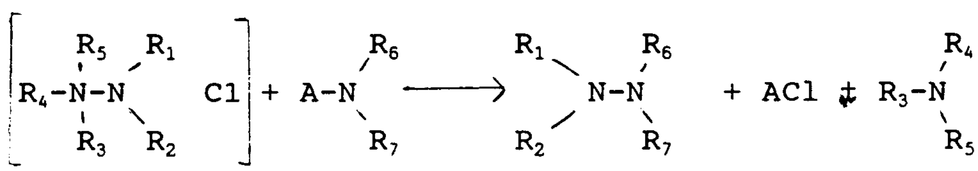

(VI) (VII)

wherein X represents and alkali metal or an alkaline earth metal; $R_1$ and $R_2$ may be the same or different and represent hydrogen or hydrocarbyl radicals; $R_3$, $R_4$ and $R_5$ may be the same or different and represent hydrocarbyl radicals; A represents an alkali metal or an alkaline earth metal; and $R_6$ and $R_7$ may be the same or different and represent hydrogen or hydrocarbyl radicals.

The proportion of reactants of formulae I and II employed in the chloramine reaction is not critical and may vary over a wide range. The stoichiometry of the reaction requires one mole of the formula I compound per mole of the formula II compound. A slight molar excess of the chlorinating agent of formular II present in the reaction mixture may be advantageous.

The chloramine reaction is preferably carried out in an inert, non-aqueous reaction medium. The relative proportion of the reaction medium to the reactants is not critical. In general, the proportion of the reaction medium is from 25 to 500 percent by weight of the reactants of the formulae I and II. It is preferred that the reaction medium be miscible with the product chloramine and the tertiary amine with which the chloramine is to be reacted in the following process step, but immiscible with water.

The reaction to form chloramine may be carried out over a wide range of temperatures, e.g. from -10 ° C to 150 ° C, and at atmospheric, subatmospheric or superatmospheric pressure. The reaction is most conveniently carried out at room temperature and atmospheric pressure. Upon completion of the reaction the pressure may be reduced to remove any unreacted volatile materials from the reaction vessel.

The order of addition of reactants is not critical. Satisfactory results have been obtained by placing the compound of formula I into a reaction vessel containing the non-aqueous, inert reaction medium and thereafter adding the chlorinating agent.

Generally, the reaction is completed within 30 to 60 minutes. Of course, the amounts of reactants employed will have an effect on the reaction time. Progress of the reaction may be monitored by employing conventional analytical instruments to determine the disappearance of reactants and the appearance of chloramine (formula III). Upon completion of the reaction, the chloramine may be separated from any by-product by conventional techniques such as distillation, decantation, freezing or by use of a precipitating agent.

The chloramine thus produced is reacted with a tertiary amine of formula IV to produce a tertiary

hydrazinium chloride of formula V (reaction B), according to the procedure of Sisler et al, Inorganic Syntheses, Vol. V, pp. 91-95. In this reaction, the chloramine is introduced into a slight excess of tertiary amine, e.g. trimethyl-, triethyl- or tripropylamine maintained at a temperature of -20 °C to -40 °C. As the reactants are mixed together, crystallization of the tertiary hydrazinium chloride begins to occur. The reaction goes to completion in 30 to 60 minutes. The reaction mixture is then allowed to warm to room temperature, washed with a suitable solvent (e.g. kerosene) to remove any residual tertiary amine, filtered and dried. The yield of tertiary-hydrazinium salt is quantitative and the overall yield for the first two steps of the reaction scheme is 65% or better, based on the amount of the formula I compound.

Tertiary hydrazinium chlorides, such as trimethylhydrazinium chloride, tripropylhydrazinium chloride, tri-n-heptylhydrazinium chloride, dimethylphenylhydrazinium chloride, dimethyl-p-tolylhydrazinium chloride, cyclohexyldiethylhydrazinium chloride tripropylmonomethylhydrazinium chloride, and tripropyldimethylhydrazinium chloride are readily prepared using the procedure of Sisler et al.

The reactions to form chloramine and tertiary hydrazinium chloride may be carried out step-wise or simultaneously in a common reaction vessel.

The preparation of anhydrous hydrazine or hydrocarbyl-substituted hydrazine by reacting the tertiary hydrazinium chloride thus produced with an alkali metal amide, an alkaline earth metal amide, a hydrocarbyl-substituted alkali metal amide or a hydrocarbyl-substituted alkaline earth metal amide in the presence of a non-aqueous reaction medium (reaction C) is set forth in detail in my aforementioned U.S. Patent No. 4,286,108, the entire disclosure of which is incorporated herein by reference, as if written out in full herein.

If desired, mixtures of hydrocarbyl-substituted hydrazines, such as a mixture of monomethylhydrazine and dimethylhydrazine, may be prepared in a common reaction and separated by techniques well known in the art, e.g. fractional distillation. This may be accomplished by chlorinating a mixture of mono- and dimethylamine to form a mixture of mono- and dimethylchloramine, which is thereafter reacted with a tertiary amine and a compound selected from an alkali metal amide, an alkaline earth metal amide, a hydrocarbyl-substituted alkali metal amide, or a hydrocarbyl-substituted alkaline earth metal amide, in succession, to produce the desired mixture of products.

As those skilled in the art will recognize, the process of the present invention may be carried out batch-wise, or, preferably, as a continuous process.

The following examples describe the manner and process of making and using the invention as set forth the best mode contemplated by the inventor for carrying out the invention.

EXAMPLE 1

A gas washing bottle provided with a gas dispersion tube at the bottom was charged with 100 ml. of kerosene, 0.1 mole of sodamide (4.0 grams) and 0.1 mole of tripropylamine (14.4 grams). A 0.1 mole quantity of chlorine gas (7.1 grams) diluted with nitrogen gas was introduced into the reaction mixture through the dispersion tube. The reaction was carried out at 0 °C for 30-40 minutes. Thereafter, the temperature of the reaction mixture was allowed to come to room temperature and the pressure in the reaction vessel was reduced to remove any volatile material. The yield of 1,1,1-tripropylhydrazinium chloride which contained some sodium chloride, as well, was 14.8 g.

The 1,1,1-tripropylhydrazinium chloride was reacted with sodamide to produce anhydrous hydrazine.

A 100 ml. three-necked reaction vessel was fitted with an efficient mechanical stirrer, an addition funnel, a sub-surface gas inlet tube, a distillation vapor thermometer, and a 20 cm. glass condenser set downward for distillation. The condenser was connected in series, to an oil-filled bubbler, a liquid trap containing sulfuric acid, a second oil-filled bubbler, and finally a gas trap cooled to -78 °C with a dry ice-acetone bath, and attached to a receiver cooled in a dry-ice acetone bath.

Fifty milliliters of kerosene (Fisher Scientific Co.), which was dried by refluxing over sodium metal, and 0.1 mole of sodamide (Fisher Scientific Co.) were heated in the three-decked reaction vessel to 150 °C under a gentle steam of nitrogen gas. Heating was continued until there was no further evolution of ammonia. Approximately 0.1 mole of dry 1,1,1-Tripropylhydrazinium chloride was slurried in 20 ml. of dry kerosene and placed in the dropping funnel.

The salt slurry was added to the three-necked reaction vessel during a 10 minute period. A white vapor was noted and the vapor temperature increased to 100 °C during the salt addition. After 7 minutes the formation of white vapor ceased. The reaction vessel temperature was increased to 165 °C and distillation proceeded for 30 minutes.

After distillation, the fraction collected was distilled using a fractionating column, yielding pure anhydrous hydrazine. The yield of anhydrous hydrazine was 39.6% (determined as the average of three runs)

based on the initial amount of sodamide used to form the chloramine.

EXAMPLE 2

Monomethylhydrazine was produced by repeating the procedure of Example 1, but substituting 1,1,1-tripropylmethylhydrazinium chloride for the 1,1,1-tripropylhydrazinium chloride. The yield of monomethylhydrazine was 53.2% (determined as the average yield of fifteen runs) based on the amount of amine used in forming the chloramine.

EXAMPLE 3

UDMH was produced by repeating the procedure of Example 1, but substituting 1,1,1-tripropyldimethylhydrazinium chloride for the 1,1,1-tripropylhydrazinium chloride. The yield of UDMH was 56.9% (determined as the average of sixteen runs) based on the amount of the amine used in forming the chloramine.

The hydrazine and hydrocarbyl-substituted hydrazines produced in Examples 1 to 3 are readily recovered as anhydrous products, with considerably less distillation than is required in prior art processes.

The products produced in Examples 1, 2 and 3 were identified by boiling point and density measurements, the measured values corresponding closely to those given in the chemistry literature for anhydrous hydrazine (b.p.113.8 °C; d. 1.004), methylhydrazine (b.p. 87.5 °C; d. 0.874), and UDMH (b.p. 63.9 °C; d. 0.791).

The procedure for producing anhydrous hydrazine described in Examples 1 and 2 produces sodium chloride and tripropylamine as by-products. The tripropylamine is recoverable in almost quantitative yields and may be conveniently recirculated to provide starting material for the tertiary hydrazinium chloride reaction. The sodium chloride may be subjected to electrolysis to produce sodium, which may be reacted with ammonia to provide sodamide (generating one-half a mole of hydrogen as a by-product, which may be recovered), and chlorine, which may be used in the reaction to form chloramine. By proceeding in this manner, the operating cost of the process may be significantly reduced. Indeed, the cost of the alkali metal amide may be viewed, more or less, as a capital expenditure, since it is continuously regenerated in the process. As previously mentioned, the process may be conveniently run using either sodamide or lithium amide.

Furthermore, the overall process for producing anhydrous hydrazine according to the present invention has a most favorable heat balance, producing about 5.0 million kcal./day*(as indicated in the Heats of Reaction table below).

HEATS OF REACTION

| | | |
|---|---|---|
| $2\ Na + 2\ NH_3$ | $\longrightarrow\ 2\ NaNH_2 + H_2$ | 1,187,500 KCal./day* |
| $Cl_2 + NaNH_2$ | $\longrightarrow\ NaCl + ClNH_2$ | 1,317,188 |
| $ClNH_2 + R_3N$ | $\longrightarrow\ R_3NClNH_2$ | 531,250 |
| $R_3NClNH_2 + NH_2 + NaNH_2$ | $\longrightarrow\ R_3N + NaCl + N_2H_4$ | 2,045,312 |

* - Values based on an assumed production of 1000 kg of anhydrous hydrazine per day.

Of course, the electrical energy required for production of sodium and chlorine must be considered in determining the overall efficiency of the process, but this requirement can be offset by the energy available from the combustion of the hydrogen generated during the formation of the alkali metal amide.

Although the above examples relating to the preparation of hydrocarbyl-substituted hydrazines are directed to the preparation of alkyl-substituted hydrazines, the same general procedure may be followed for the preparation of aryl-, aralkyl- and cycloalkyl- substituted hydrazines. For example, the sodium derivatives of aniline, benzylamine or cyclohexylamine may be prepared and reacted with an appropriate tertiary-hydrazinium salt to produce the corresponding hydrocarbyl-substituted product, i.e., phenylhydrazine etc., as described in US Patent No 4,286,108.

* - Values based on an assumed production of 1000 kg of anhydrous hydrazine per day.

## Claims

1. A process for preparing hydrazine and hydrocarbyl-substituted hydrazines under anhydrous reaction conditions which comprises:
   a. reacting a compound of the formula X-$NR_1R_2$, wherein X is selected from alkali metal or an alkaline earth metal and $R_1$ and $R_2$ may be the same or different and are selected from hydrogen and hydrocarbyl radicals, with chlorine effective to produce a chloramine of the formula C1-$NR_1R_2$, wherein $R_1$ and $R_2$ are as previously defined, without co-production of ammonium chloride;
   b. reacting said chloramine with an at least equimolar amount of a tertiary amine of the formula $NR_3R_4R_5$, wherein $R_3$, $R_4$ and $R_5$ may be the same or different and represent hydrocarbyl radicals, to produce a tertiary hydrazinium chloride of the formula $NR_1R_2NR_3R_4R_5C1$, wherein $R_1$, $R_2$, $R_3$, $R_4$ and $R_5$ are as previously defined; and
   c. reacting said tertiary hydrazinium chloride with a compound of the formula $ANR_6R_7$, wherein A represents an alkali metal or an alkaline earth metal and $R_6$ and $R_7$ may be the same or different and are selected from hydrogen and hydrocarbyl radicals, to produce an anhydrous hydrazine of the formula $R_1R_2N$-$NR_6R_7$.

2. The process according to Claim 1 wherein chlorine is reacted with an alkali metal amide.

3. The process according to Claim 2 wherein the alkali metal amide is sodamide or lithium amide.

4. The process according to Claim 3 wherein the reaction medium is dry kerosene.

5. A process for preparing hydrazine under anhydrous reaction conditions comprising the steps of:
   a. electrolyzing sodium chloride or lithium chloride to give sodium or lithium and chlorine;
   b. reacting the sodium or lithium resulting from said electrolysis with ammonia to produce sodamide or lithamide;
   c. reacting approximately one-half of the sodamide or lithamide produced in step b with the chorine resulting from said electrolysis to produce chloramine and sodium chloride or lithium chloride;
   d. reacting said chloramine with an at least equimolar amount of a tertiary-hydrocarbylamine to produce a tertiary-hydrocarbyl hydrazinium chloride; and
   e. reacting said tertiary-hydrocarbyl hydrazinium chloride with the remaining amount of sodamide or lithamide produced in step b under anhydrous conditions to produce a tertiary-hydrocarbylamine, sodium chloride or lithium chloride and anhydrous hydrazine.

6. The process according to Claim 5 wherein said hydrazine product is separated from the reaction mixture by distillation.

7. The process according to Claim 5 wherein the sodium chloride produced in steps c and e are recirculated to provide the starting material for the electrolysis step.

8. The process according to Claim 5 wherein the tertiary-hydrocarbylamine produced in step e is recirculated to provide starting material for step d.

## Revendications

1. Un procédé de préparation d'hydrazine et d'hydrazines hydrocarbyl-substituées dans des conditions anhydres de réaction, qui comprend les étapes consistant à :
   a. faire réagir un composé de formule X-$NR_1R_2$, dans laquelle X est choisi parmi un métal alcalin ou un métal alcalinoterreux et $R_1$ et $R_2$ peuvent être identiques ou différents et sont choisis parmi l'hydrogène et des radicaux hydrocarbyl, avec le chlore pour produire une chloramine de la formule Cl-$NR_1R_2$, dans laquelle $R_1$ et $R_2$ sont comme définis précédemment, sans coproduction de chlorure d'ammonium ;
   b. faire réagir ladite chloramine avec une quantité au moins équimolaire d'une amine tertiaire de la formule $NR_3R_4R_5$, dans laquelle $R_3$, $R_4$ et $R_5$ peuvent être identiques ou différents et représentent des radicaux hydrocarbyl, pour produire un chlorure d'hydrazinium tertiaire de formule $NR_1R_2NR_3R_4R_5Cl$, dans laquelle $R_1$, $R_2$, $R_3$, $R_4$ et $R_5$ sont définis comme précédemment ; et
   c. faire réagir ledit chlorure d'hydrazinium tertiaire avec un composé de formule $ANR_6R_7$ dans

laquelle A représente un métal alcalin ou un métal alcalino-terreux et $R_6$ et $R_7$ peuvent être identiques ou différents et sont choisis parmi l'hydrogène et des radicaux hydrocarbyl, afin de produire une hydrazine anhydre de formule $R_1R_2N\text{-}NR_6R_7$.

**2.** Le procédé selon la revendication 1 dans lequel le chlore est mis en réaction avec un amide d'un métal alcalin.

**3.** Le procédé selon la revendication 2 dans lequel l'amide de métal alcalin est l'amide de sodium ou de lithium.

**4.** Le procédé selon la revendication 3 dans lequel le milieu de réaction est du kérosène sec.

**5.** Un procédé de préparation d'hydrazine dans des conditions anhydres de réaction comprenant les étapes consistant à :
a. électrolyser du chlorure de sodium ou du chlorure de lithium pour donner du sodium ou du lithium et du chlore ;
b. faire réagir le sodium ou le lithium résultant de ladite électrolyse avec de l'ammoniac gazeux pour produire l'amide de sodium ou de lithium ;
c. faire réagir approximativement une moitié de l'amide de sodium ou de lithium produit à l'étape b avec le chlore résultant de ladite électrolyse pour produire de la chloramine, et du chlorure de sodium ou de lithium ;
d. faire réagir ladite chloramine avec une quantité au moins équimolaire d'une hydrocarbylamine tertiaire pour produire un chlorure d'hydrocarbyl-hydrazinium tertiaire ; et
e. faire réagir dans des conditions anhydres ledit chlorure d'hydrocarbyl-hydrazinium tertiaire avec la quantité restante d'amide de sodium ou de lithium produite à l'étape b pour produire une hydrocarbylamine tertiaire, du chlorure de sodium ou de lithium et de l'hydrazine anhydre.

**6.** Le procédé selon la revendication 5, dans lequel ledit produit hydrazine est séparé du mélange de réaction par distillation.

**7.** Le procédé selon la revendication 5, dans lequel le chlorure de sodium produit aux étapes c et e est remis en circulation pour constituer la matière première de l'étape d'électrolyse.

**8.** Le procédé selon la revendication 5, dans lequel l'hydrocarbylamine tertiaire produite à l'étape e est remise en circulation pour constituer la matière première de l'étape d.

## Patentansprüche

**1.** Verfahren zur Herstellung von Hydrazin und Hydrocarbyl-substituierten Hydrazinen bei wasserfreien Reaktionsbedingungen, dadurch **gekennzeichnet,** daß man
a. eine Verbindung der Formel $X\text{-}NR_1R_2$, worin X ein Alkalimetall oder ein Erdalkalimetall bedeutet und $R_1$ und $R_2$, die gleich oder unterschiedlich sein können, Wasserstoff oder Hydrocarbylgruppen bedeuten, mit Chlor unter Bildung des Chloramins der Formel $Cl\text{-}NR_1R_2$ umsetzt, worin $R_1$ und $R_2$ die zuvor gegebenen Definitionen besitzen, ohne daß gleichzeitig Ammoniumchlorid gebildet wird;
b. das Chloramin mit mindestens der äquimolaren Menge eines tertiären Amins der Formel $NR_3R_4R_5$, worin $R_3$, $R_4$ und $R_5$, die gleich oder unterschiedlich sein können, Hydrocarbylgruppen bedeuten, unter Bildung eines tertiären Hydraziniumchlorids der Formel $NR_1R_2NR_3R_4R_5Cl$ umsetzt, worin $R_1$, $R_2$, $R_3$, $R_4$ und $R_5$ die zuvor gegebenen Bedeutungen besitzen; und
c. das tertiäre Hydraziniumchlorid mit einer Verbindung der Formel $ANR_6R_7$, worin A ein Alkalimetall- oder ein Erdalkalimetall bedeutet und $R_6$ und $R_7$, die gleich oder unterschiedlich sein können, Wasserstoff oder Hydrocarbylgruppen bedeuten, unter Bildung des wasserfreien Hydrazins der Formel $R_1R_2N\text{-}NR_6R_7$ umsetzt.

**2.** Verfahren nach Anspruch 1, dadurch **gekennzeichnet,** daß man Chlor mit einem Alkalimetallamid umsetzt.

**3.** Verfahren nach Anspruch 2, dadurch **gekennzeichnet,** daß man als Alkalimetallamid Natriumamid oder Lithiumamid verwendet.

4. Verfahren nach Anspruch 3, dadurch **gekennzeichnet,** daß man als Reaktionsmedium trockenes Kerosin verwendet.

5. Verfahren zur Herstellung von Hydrazin bei wasserfreien Reaktionsbedingungen, dadurch **gekennzeichnet,** daß man
   a. Natriumchlorid oder Lithiumchlorid unter Bildung von Natrium oder Lithium und Chlor elektrolysiert;
   b. das Natrium oder Lithium, das bei der Elektrolyse gebildet wird, mit Ammoniak unter Bildung von Natriumamid oder Lithiumamid umsetzt;
   c. ungefähr die Hälfte des Natriumamids oder Lithiumamids, das bei der Stufe b. gebildet wird, mit dem Chlor, das bei der Elektrolyse gebildet wird, unter Bildung von Chloramin und Natriumchlorid oder Lithiumchlorid umsetzt;
   d. das Chloramin mit mindestens der äquimolaren Menge eines tertiären Hydrocarbylamins unter Bildung eines tertiären Hydrocarbylhydraziniumchlorids umsetzt; und
   e. das tertiäre Hydrocarbylhydraziniumchlorid mit der restlichen Menge an Natriumamid oder Lithiumamid, das bei der Stufe b. gebildet wurde, bei wasserfreien Bedingungen unter Bildung eines tertiären Hydrocarbylamins, Natriumchlorids oder Lithiumchlorids und wasserfreien Hydrazins umsetzt.

6. Verfahren nach Anspruch 5, dadurch **gekennzeichnet,** daß das Hydrazinprodukt von dem Reaktionsgemisch durch Destillation abgetrennt wird.

7. Verfahren nach Anspruch 5, dadurch **gekennzeichnet,** daß das bei den Stufen c. und e. gebildete Natriumchlorid recyclisiert wird und als Ausgangsmaterial bei der Elektrolysestufe verwendet wird.

8. Verfahren nach Anspruch 5, dadurch **gekennzeichnet,** daß das bei der Stufe e. gebildete tertiäre Hydrocarbylamin recyclisiert wird und als Ausgangsmaterial bei der Stufe d. verwendet wird.